(19) Europäisches Patentamt
European Patent Office
Office européen des brevets

(11) **EP 1 764 430 A1**

(12) **EUROPEAN PATENT APPLICATION**
published in accordance with Art. 158(3) EPC

(43) Date of publication:
**21.03.2007 Bulletin 2007/12**

(21) Application number: **05753335.8**

(22) Date of filing: **27.06.2005**

(51) Int Cl.:
*D04B 1/16* (2006.01)   *A41B 9/00* (2006.01)
*A41B 11/00* (2006.01)   *A41B 17/00* (2006.01)
*A61L 15/00* (2006.01)   *D01F 8/10* (2006.01)
*D03D 15/00* (2006.01)

(86) International application number:
**PCT/JP2005/011753**

(87) International publication number:
**WO 2006/008916 (26.01.2006 Gazette 2006/04)**

(84) Designated Contracting States:
**AT BE BG CH CY CZ DE DK EE ES FI FR GB GR HU IE IS IT LI LT LU MC NL PL PT RO SE SI SK TR**

(30) Priority: **02.07.2004 JP 2004196430**

(71) Applicant: **Kuraray Co., Ltd.,**
**Kurashiki Plant**
**Okayama 710-8622 (JP)**

(72) Inventors:
• **KAWAMOTO, Masao**
**c/o Kuraray Co. Ltd.**
**Okayama 7108622 (JP)**

• **TANAKA, Kazuhiko**
**c/o Kuraray Co. Ltd.**
**Osaka-shi**
**Osaka 530-8611 (JP)**
• **AMIYA, Shigetoshi**
**c/o Kuraray Co. Ltd.**
**Osaka-shi**
**Osaka 530-8611 (JP)**
• **ARUGA, Mitsotake**
**c/o Kuraray Trading Co. Ltd.**
**Osaka-shi**
**Osaka 530-8611 (JP)**

(74) Representative: **Vossius & Partner**
**Siebertstrasse 4**
**81675 München (DE)**

(54) **FABRIC AND CLOTHES FOR ATOPIC DERMATITIS PATIENTS**

(57)   To provide a fabric and clothing having a low irritation to patients with atopic dermatitis with high productivity. The fabric is produced as a fabric for patients with atopic dermatitis, which is contactable with an affected area of patients with atopic dermatitis and comprises an ethylene-vinyl alcohol (EVOH)-series fiber comprising an ethylene-vinyl alcohol-series copolymer having an ethylene content of 25 to 70% by mole, in which the surface of the EVOH-series fiber is occupied by the ethylene-vinyl alcohol-series copolymer in a proportion of not less than 70%, wherein the hydroxyl group amount of the EVOH-series fiber surface is 20 to 70% by mole, the Young's modulus of the EVOH-series fiber is not more than 60 cN/dtex, and the single fiber fineness of the EVOH-series fiber is not more than 5 dtex. The EVOH-series fiber may be a sheath-core structure conjugated fiber comprising the EVOH-series copolymer as the sheath component, in particular a sheath-core structure conjugated fiber which comprises the EVOH-series copolymer having an ethylene content of 25 to 70% by mole as the sheath component, and a crystalline thermoplastic resin having a melting point of not lower than 150°C as the core component.

F I G. 1

**Description**

TECHNICAL FIELD

The present invention relates to a fabric and a clothing which comprise a synthetic fiber having a sufficient physical properties as a fiber and being excellent in durability or fabric-forming property, as well as which are suitable for alleviating (or relieving) itching (or pruritus) from an affected area of a patient with atopic dermatitis or for treating (healing or curing) a scratched skin lesion associated with the itching (or pruritus).

BACKGROUND ART

Heretofore, as a fiber material excellent in biocompatibility, there have been known fibers obtained from a chitin which is a natural polymer substance contained in an exoskeleton of crab, shrimp or the like, or a chitosan which is a deacetylated product of the chitin. For example, Japanese Application Laid-Open No.92820/1996 (JP-8-92820A, Patent Document 1) discloses that in the case where a chitin is subjected to viscose process, and a mixed liquid of the viscose chitin and a viscose cellulose is used as a spinning stock solution, a fiber can be produced by a wet spinning method adopted for a common viscose rayon. Moreover, "The preparation and application of functional fibers from crab shell chitin" (Journal of Biotechnology, 70, (1999) 373-377) (Nonpatent Document 1) mentions an artificial filament obtained by blending a chitin and a silk fibroin.

However, these techniques have had some problems as follows: a blend fiber obtained by blend-spinning a chitin or chitosan and other fiber lacks in a sufficient strength as a fiber material when the content of the chitin or chitosan is increased. Moreover, in the techniques of Patent Document 1, use of poisonous carbon disulfide in xanthate process of the chitin has an adverse affect on human body or environment. Further, in the above-mentioned techniques, since the chitin or chitosan is blend-spun with other fiber, 100% of the surface of thus obtained fiber is not necessarily covered with the chitin or chitosan. Therefore, there has been room for improvement in the light of achieving the aimed effect. Moreover, other functions such as hygroscopicity or dye-affinity are also insufficient.

On the other hand, in order to ensure a fiber strength, there has been proposed to coat a cellulose fiber with a chitin or chitosan. However, this technique has a problem that the chitin or chitosan is peeled off from the coated fiber in use or repeated washing, as a result such a fiber is poor in durability.

Further, it is proposed to use a natural silk. Since the silk has extremely excellent luster and flexible feel, as well as superb absorption and desorption of moisture, the silk is regarded as an excellent material which is peerless as a high-end clothing material. Moreover, the silk has been expanded in application such as a medical material (e.g., a suture thread) or a cosmetic material due to its biocompatibility. However, since the silk is limited in the amount supplied and is extremely comparatively high in price, the silk supplies in disproportion with the need in the present circumstances.

From such a circumstance, recently, the research and development for obtaining an alternative material (such as a fiber or a film) having properties extremely close to a silk are energetically pushed forward, and some techniques are proposed. More specifically, there have been proposed a process which comprises chemically modifying a surface of a synthetic fiber or film with a silk protein-containing finishing liquid to impart silk properties to the synthetic fiber or film, a process which comprises allowing a silk powder to be involved in a thermoplastic synthetic resin to impart silk properties to a molded product of the thermoplastic synthetic polymer, and others [for example, Japanese Patent Application Laid-Open No. 212456/1998 (JP-10-212456A, Patent Document 2), and Japanese Patent Application Laid-Open No.44598/2000 (JP-2000-44598A, Patent Document 3)].

In these processes, however, while the properties of the silk protein can be generally imparted to the surface of the material, due to use of an aqueous solution or dispersion of the silk protein as a finishing agent, the silk protein tends to be peeled off in use or repeated washing in the same as the above-mentioned process. As a result, these processes have a shortcoming of being poor in durability of the resulting matter. The following process may be also considered: preparing a mixture of a silk protein and a binder or a coating agent (or a paint and varnish) as a finishing liquid, and coating a surface of a fiber or a fabric with the finishing liquid. However, in the case of this process, the surface of the silk protein is coated with the binder or the coating resin, and therefore, a problem that the properties of the silk protein cannot be sufficiently exhibited arises.

As a process for solving this problem, there is a process proposed in Japanese Patent Application Laid-Open No. 342835/2003 (JP-2003-342835A, Patent Document 4), that is, a process which comprises kneading a silk particle with a thermoplastic resin having an appropriate melting point, and using the kneaded resin as a sheath component to make a sheath-core structure conjugated spinning fiber. However, in the same manner as the above-mentioned process, even in the case of this process the desired object cannot be accomplished because the silk protein is coated with the resin. Further, there has been a problem that the species of the thermoplastic resin to be used is extremely limited.

In particular, recently, the number of patients with atopic dermatitis has been increased sharply due to change of living environment. In the case of wearing underwear made of a common synthetic fiber(s), atopic dermatitis tends to take a turn for the worse. Even in the case of wearing underwear made of a natural cotton, a sheep wool, or other natural one, the patients cannot be rewarded with the desired result.

On the other hand, Japanese Patent Application Laid-Open No. 213535/1991 (JP-3-213535A, Patent Document 5) discloses a textile, as a clothing such as an inner wear, an intermediate garment or a sportswear, which comprises a multi-component fiber having a fineness of single fiber of not more than 5 denier, wherein the fiber comprises (A) a saponified polymer of an ethylene-vinyl acetate copolymer having an ethylene content of 30 to 70% by mole and (B) a thermoplastic polyester-series polymer in a mass ratio (A: B) of 5:95 to 60 : 40 , and which has a specific relationship between a specific coefficient of moisture absorption and a specific speed for absorbing and diffusing water. This document, however, fails to describe biocompatibility of fiber, particularly, relationship between a disease such as atopic dermatitis and fiber.

Further, "Study on affectivity of a coefficient of variation of perspiration differential amount as an evaluation index of biocompatibility of various fibers" (Japanese Journal of Perspiration Research, 2002, Vol.9 No . 2 , pp. 57 - 60) (Nonpatent Document 2) describes the result of a research into the effect of a polyethylene-vinyl alcohol fiber on mental sweating of a patient with atopic dermatitis as a trial subject. However, this document does not disclose details of the employed polyethylene-vinyl alcohol, and never describes the relationship between the characteristic of the polyethylene-vinyl alcohol and the atopic dermatitis.

Patent Document 1: JP-8-92820A
Patent Document 2: JP-10-212456A
Patent Document 3: JP-2000-44598A
Patent Document 4: JP-2003-342835A
Patent Document 5: JP-3-213535A (Claims, page 1, left-hand column, line 17 to right-hand column, line 3, and Examples)
Nonpatent Document 1: "The preparation and application of functional fibers from crab shell chitin" (Journal of Biotechnology 70 (1999) 373-377)
Nonpatent Document 2: "Study on affectivity of a coefficient of variation of perspiration differential amount as an evaluation index of biocompatibility of various fibers" (Japanese Journal of Perspiration Research, 2002, Vol.9 No. 2, pp. 57-60)

DISCLOSURE OF THE INVENTION

PROBLEMS TO BE SOLVED BY THE INVENTION

It is therefore an object of the present invention to provide a fabric and a clothing having a low (or mild) irritation to patients with atopic dermatitis and being high in productivity.

It is another object of the present invention to provide a fabric and a clothing which are excellent in physical properties of a fiber as well as in fabric-formability and are capable of alleviating or healing (or curing) dermatitis symptoms of atopic dermatitis.

It is still another object of the present invention to provide a fabric and a clothing hardly deteriorating in effects of alleviating or healing (or curing) atopic dermatitis even with repeated washing.

MEANS TO SOLVE THE PROBLEMS

The inventors of the present invention made intensive studies and finally found that a fabric formed from a specific ethylene-vinyl alcohol-series (EVOH-series) fiber realizes high productivity as well as low irritation even in contacting with an affected area of patients with atopic dermatitis.

That is, the fabric of the present invention is a fabric for patients with atopic dermatitis, which is contactable with an affected area of patients with atopic dermatitis and comprises an ethylene-vinyl alcohol (EVOH)-series fiber comprising an ethylene-vinyl alcohol-series copolymer having an ethylene content of 25 to 70% by mole, in which the surface of the EVOH-series fiber is occupied by the ethylene-vinyl alcohol-series copolymer in a proportion of not less than 70%, wherein the proportion (or mole proportion) of the vinyl alcohol unit existing on the EVOH-series fiber surface is 20 to 70% by mole, the Young's modulus of the EVOH-series fiber is not more than 60 cN/dtex, and the single fiber fineness of the EVOH-series fiber is not more than 5 dtex. Incidentally, the term "the proportion (or mole proportion) of the vinyl alcohol unit existing on the EVOH-series fiber surface" is hereinafter sometimes simply referred to as "hydroxyl group amount on the fiber surface", "hydroxyl group amount" or "OH group concentration". Moreover, the hydroxyl group amount on the fiber surface means a proportion of a vinyl alcohol unit having a free hydroxyl group, and is practically measured as a lower value than a theoretical value. Accordingly, the hydroxyl group amount means not the theoretical value, but the practically measured value with an X-ray photoelectron spectroscopy (XPS). The hydroxyl group amount can be measured, for example, with "AXIS-HSi" manufactured by Shimadzu Corporation. The EVOH-series fiber may be a sheath-core structure conjugated (or composite) fiber comprising the ethylene-vinyl alcohol-series copolymer as the sheath component, in particular a sheath-core structure conjugated fiber which comprises the ethylene-vinyl alcohol-series copolymer having an ethylene content of 25 to 70% by mole as the sheath component, and a crystalline thermoplastic resin having a melting point of not lower than 150°C as the core component. Such a sheath-core structure conjugated fiber, for example, may comprise a core having a cross-sectional structure having not less than 4 protrusions in a perpendicular direction to the longitudinal direction of the fiber and may have a ratio of a peripheral length of the core (L2) relative to a peripheral length of the conjugated fiber (L1) satisfying the following formula (1):

$$1.6 \leq X/C \qquad (1)$$

wherein, X represents a ratio (L2/L1) of the peripheral length of the core (L2) relative to the peripheral length of the conjugated fiber (L1), and C represents the conjugated ratio (mass ratio) of the core relative to the conjugated fiber when the mass of the conjugated fiber is taken as 1.

The sheath component may further comprise, in a proportion of 5 to 45% by mass relative to the sheath component, a polyamide resin whose terminal amino group is at least partly capped. Moreover, the EVOH-series fiber may constitute a multifilament yarn having a whole fineness of 40 to 160 dtex, the multifilament yarn may be false-twisted, and the false-twisted yarn may be further actual-twisted at a twist number of 500 to 1500 T/m. The ethylene-vinyl alcohol-series copolymer may contain an alkali metal ion and an alkaline earth metal ion in a proportion of not more than 100 ppm on the mass basis, respectively, and the EVOH-series fiber may contain an oily substance comprising a nonionic surfactant. Moreover, in the fabric of the present invention, an area contacting with an affected area of a wearer may comprise the EVOH-series fiber.

The present invention also includes a clothing for patients with atopic dermatitis, which comprises the above-mentioned fabric. The clothing may encompass an underwear, a shirt, a sleeping wear, a hosiery, gloves (mittens or gauntlets), a mask, and others. Such a clothing may be an underwear for patients with atopic dermatitis, which comprises at least the EVOH-series fiber on the side (surface) contacting with the skin of the patient and at least a cellulose-series fiber on the other side thereof.

The present invention also embraces a method for alleviating (mitigating) or healing (curing) a dermatitis symptom (eczema) of patients with atopic dermatitis with the use of the fabric.

Incidentally, in the present description, the ethylene-vinyl alcohol-series copolymer is sometimes simply referred to as "polymer A", and in the case of the conjugated fiber, a polymer (particularly a crystalline thermoplastic polymer) which is conjugated with the polymer A is sometimes simply referred to as "polymer B".

EFFECTS OF THE INVENTION

According to the present invention, since the fabric comprises a specific ethylene-vinyl alcohol-series fiber, the fabric is high in productivity, and has a low irritation to patients with atopic dermatitis. This fabric is excellent in physical properties of a fiber and in fabric-formability, as well as is capable of alleviating or healing dermatitis symptoms of atopic dermatitis. This fabric hardly deteriorates in effects of alleviating or healing atopic dermatitis even in repeated washing. Such a fabric is suitable for a clothing such as an underwear, a shirt, a sleeping wear, a hosiery, gloves, or a mask. That is, the clothing formed from the fabric of the present invention is excellent in biocompatibility with human body, and in the case where a patient with atopic dermatitis wears the clothing, the degree of itching or scratched lesion is decreased due to the clothing having a low irritation to skin. Thereby the clothing (fabric) is excellent in effects of alleviation or healing (curing) of atopic symptoms.

BRIEF DESCRIPTION OF DRAWINGS

Fig. 1 is a cross-sectional microphotograph (5000 magnifications) illustrating an embodiment of the sheath-core structure conjugated fiber of the present invention.

Fig. 2 is a schematic cross-sectional view illustrating another embodiment of the sheath-core structure conjugated fiber of the present invention.

Fig. 3 is a schematic cross-sectional view illustrating still another embodiment of the sheath-core structure conjugated fiber of the present invention.

Fig. 4 is a schematic cross-sectional view illustrating a further embodiment of the sheath-core structure conjugated fiber of the present invention.

DETAILED DESCRIPTION OF THE INVENTION

[EVOH-series fiber]

The fabric for patients with atopic dermatitis of the present invention is a fabric which is contactable with an affected area of patients with atopic dermatitis and comprises an EVOH-series fiber comprising an ethylene-vinyl alcohol-series copolymer having an ethylene content of 25 to 70% by mole, in which the surface of the EVOH-series fiber is occupied by the copolymer in a proportion of not less than 70% (hereinafter, the copolymer sometimes referred to as "polymer A").

(Polymer A)

The polymer A means a copolymer comprising an ethylene unit and a vinyl alcohol unit as a base skeleton, and obtainable by saponification of an ethylene-vinyl acetate copolymer. The saponification degree needs to be as high as not less than 95% by mole, and the content (copolymerization proportion) of the ethylene unit in the base skeleton needs to be about 25 to 70% by mole (particularly about 30 to 65% by mole). Therefore, the proportion of the vinyl alcohol unit [including a copolymerization unit in which OH group of the vinyl alcohol unit is OR group (R is an organic group) due to unsaponification of the ethylene-vinyl acetate copolymer or others] is most suitably about 30 to 75% by mole (particularly about 35 to 70% by mole). Too low content of the vinyl alcohol unit in the polymer A is not preferred, because decrease of the number of hydroxyl groups lowers the biocompatibility of the fiber and induces strong hydrophobicity, thereby the adaptability of the fiber to skin deteriorates. On the contrary, too high content of the vinyl alcohol unit is not also preferred because, as described later, the biocompatibility of the fiber deteriorates and the spinnability thereof becomes poor, thereby single fiber tearing or fiber breakage frequently occurs in the spinning or stretching process. In the case where the content of the vinyl alcohol unit becomes too high, although the reason of lowering biocompatibility is unknown, there is a fact, for example, that a polyvinyl alcohol-series fiber, i.e. , a fiber having almost 100% by mole of the vinyl alcohol unit is inferior to an EVOH-series-copolymer-series fiber by far in terms of biocompatibility. Therefore, it is thought that coexistence of a hydrophobic ethylene unit and a hydrophilic vinyl alcohol unit in a specific proportion realizes effects of alleviating and healing itching of atopic dermatitis.
Incidentally, the ethylene-vinyl acetate copolymer may further comprise, as a copolymerizable component, (meth) acrylic acid or a derivative thereof [e.g., (meth) acrylic acid or a salt thereof, and a $C_{1-6}$ alkyl ester of (meth)acrylic acid such as methyl (meth)acrylate], (meth)acrylamide or a derivative thereof, an unsaturated carboxylic acid (e.g., maleic acid or maleic anhydride), and others.
As an example illustrating a production process of the polymer A, the process comprises radical polymerizing ethylene and vinyl acetate in a polymerization solvent such as methanol in the presence of a radical polymerization catalyst to give an ethylene-vinyl acetate-series copolymer, removing unreacted monomers from the reaction mixture, and saponifying the ethylene-vinyl acetate -series copolymer with caustic soda to give an ethylene-vinyl alcohol-series copolymer, and further pelletizing the ethylene-vinyl alcohol-series copolymer in water, rinsing the pellet with water and drying the rinsed pellet to give a polymer A.
Because of such a process, thus obtained polymer A inevitably contains an alkali metal ion or an alkaline earth metal ion. However, it is preferred that the polymer A which directly comes in contact with human skin has as high purity as possible to minimize skin irritation by impurities. That is, each of the contents of the alkali metal ion and alkaline earth metal ion in the polymer A is, on the mass basis, not more than 100 ppm (e.g., about 0.01 to 100 ppm), preferably not more than 50 ppm (e.g., about 0.01 to 50 ppm), and more preferably not more than 10 ppm (e.g. , about 0.01 to 10 ppm) . Incidentally, decrease of the contents of these metal ions not only alleviates skin irritation but also improves spinnability.
As the method for improving the purity of the polymer A, there is mentioned a method which comprises pelletizing a saponifiedmatter in the production process of the polymer, rinsing the resulting wet pellet with a large amount of a mixture containing purified water and an acid such as acetic acid, and further rinsing the rinsed matter only with an excessive amount of purified water. Existence of the alkali metal ion and/or alkaline earth metal ion in large quantity in the fiber is responsible for further itching to patients with atopic dermatitis having a weak (delicate) skin.
As described above, the polymer A is producible by saponification of an ethylene-vinyl acetate copolymer with caustic soda, and the saponification degree is preferably not less than 95% by mole (e.g., about 95 to 99.99%, and particularly about 96 to 99.9%). Too low saponification degree decreases crystallinity of the polymer, as well as physical properties of fiber such as strength. Further, the polymer A is liable to be softened, resulting in the troubles occurring in the texturing or weaving process and the deterioration of physical properties of thus obtained fiber. Thereby, the clothing formed from such a fiber is not preferred because the clothing irritates an affected area of the atopic dermatitis.

In the present invention, the fiber may comprise (consist of) the polymer A alone, or may comprise the polymer A and other component(s) as far as not less than 70% of the fiber surface is covered (or coated) with the polymer A (ethylene-vinyl alcohol-series copolymer).

In particular, the ethylene-vinyl alcohol-series copolymer as the polymer A is low in hot water resistance, and, for example, in the case of immersing a fiber comprising the polymer A in hot water, there is a problem such that the fiber agglutinates or shrinks. In order to dissolve this problem, in general, there have been adopted a method of acetalizing or formalizing the ethylene-vinyl alcohol-series copolymer. The inventors of the present invention found that such a method is effective for preventing the fiber from the agglutination or shrinkage, but that an effect of healing atopic dermatitis is decreased due to decrease of hydroxyl groups on the fiber surface. Accordingly, the inventors made studies on how to give hot water resistance to the fiber without decreasing the number of hydroxyl groups on the fiber surface, and found that addition of a polyamide resin to the ethylene-vinyl alcohol-series copolymer (polymer A) is preferably adopted. The polyamide resin may include polyamide resins exemplified in the item of the polymer B mentioned below, and an aliphatic polyamide such as a polyamide 6, a polyamide 66, a polyamide 6/12 or a polyamide 12 is usually employed.

Addition of the polyamide resin to the ethylene-vinyl alcohol-series copolymer ensures to enhance hot water resistance of the fiber, but generates gelation in the melt - spinning step and lowers spinnability. In order to prevent this gelation, it is preferred to use a polyamide resin having a capped terminal amino group. In particular, as such the polyamide resin, the preferred one includes a polyamide in which most of terminal groups are amino groups, and most of such amino groups are capped. It is not particularly limited how to cap the amino group, but from the viewpoint to improve hot water resistance more completely, capping with an end-capping agent capable of forming a cyclic imide bond with the amino group is preferred. The preferred end-capping agent includes a cyclic acid anhydride, particularly an aromatic or aliphatic carboxylic anhydride (e.g., phthalic anhydride or succinic anhydride). In the case of using phthalic anhydride or succinic anhydride, phthalimide or succinimide is formed as the cyclic imide bond.

As the method to obtain a polymer having amino groups in most of the terminal groups, concretely, there may be mentioned ring-opening polymerizing a polyamide-formable monomer comprising either caprolactam or lauryllactam, or both caprolactam and lauryllactam as a main component in the presence of a molecular weight adjusting agent (particularly a diamine having 4 to 20 carbon atoms) to form a polyamide resin comprising either caprolactam or lauryllactam, or both caprolactam and lauryllactam as a main component. The given polyamide resin has a relative viscosity [$\eta r$] at 25°C of about 2 to 7 (particularly about 2 to 5), and not less than 75% of the total terminal groups in the polyamide resin is amino groups. Subsequently, by allowing terminal amino groups of the obtained polyamide resin to react with the end-capping agent which is capable of forming cyclic imide bond, a polyamide resin which has a capped terminal amino group at least in part can be obtained.

In the case of adding the small amount of the polyamide resin to the ethylene-vinyl alcohol-series copolymer, the polyamide resin exists in a dispersed island state in the ethylene-vinyl alcohol-series copolymer, and the dispersed polyamide resin has highest hot water resistance in the case where the size of one island is about 1 nm to 300 nm, preferably about 20 to 150 nm, and more preferably about 30 to 100 nm, and where the number of islands is not less than 10 per $\mu m^2$, preferably about 30 to 500 per $\mu m^2$, and more preferably about 30 to 300 per $\mu m^2$ in the cross section of the fiber (the cross section in a perpendicular direction to the longitudinal direction of the fiber).

The amount of the polyamide resin to be blended in the ethylene-vinyl alcohol-series copolymer is preferably in a range of 5 to 45% by mass, more preferably 10 to 40% by mass (particularly 10 to 30% by mass) relative to the amount of the ethylene-vinyl alcohol-series copolymer in view of hot water resistance. Too small amount of the polyamide resin causes little improving effect of hot water resistance, whereas too large amount of the polyamide resin cannot ensure the dispersed states thereof which contribute to fiber-forming process, and in some cases, the sea-island structure therebetween may be reversed, and the given fabric deteriorates in feel.

Incidentally, with respect to the number of islands of the polyamide resin and the proportion thereof, in the case of the sheath-core structure conjugated fiber, the number and the proportion correspond to those in the sheath part thereof; and in the case of the fiber (unconjugated fiber) comprising the EVOH-series fiber alone, the number and the proportion correspond to those in the fiber entirety.

As the reason why hot water resistance is improved by blending the polyamide resin in the ethylene-vinyl alcohol-series copolymer, there may be expected a crosslinking reaction between hydroxyl groups of the ethylene-vinyl alcohol-series copolymer and terminal carboxyl groups of the polyamide resin, or a crosslinking reaction between terminal amino groups of the polyamide resin and carboxyl groups of the ethylene-vinyl alcohol-series copolymer. Incidentally, the ethylene-vinyl alcohol-series copolymer obtained by copolymerizing ethylene and vinyl acetate and saponifying the copolymerized polymer usually has carboxyl groups even without copolymerizing a carboxyl group-containing monomer. The crosslinking degree is preferably a degree which realizes a hot water resistance without causing agglutination in boiled water of 95 to 125°C, and such a crosslinking degree is highly affected by the dispersed state of the polyamide resin. That is, the polyamide as the dispersed component is preferably dispersed in a certain degree in the ethylene-vinyl alcohol-series copolymer. More specifically, the polyamide is preferably in the above-mentioned state in which the island component is dispersed to prevent agglutination or excessive shrinkage between fibers upon ironing with steam or upon washing or drying.

Incidentally, the number of island components was counted from a micrograph (x100000 enlarged) of a cross section of the fiber with a transmission electron microscope. Moreover, the size of the island component was determined in the following way. From the micrograph, 20 islands were arbitrarily selected, and the first and second largest islands and the first and second smallest islands were removed therefrom. With respect to the remaining 16 islands, a diameter of a circle corresponding to each of the islands was determined, and the average diameter of the 16 circles was calculated as the size of the island component.

In the present invention, with respect to the spinnability of the ethylene-vinyl alcohol-series copolymer as the polymer A, a large content of the vinyl alcohol component in the copolymer is likely to improve blend spinnability with the polyamide resin due to shift of the melting point peak to a higher temperature, which is measured with a differential scanning calorimeter (DSC), whereas a small content of the ethylene component in the copolymer is liable to cause decrease of melt-spinning property, and too small amount of ethylene content tends to lower the biocompatibility of the copolymer. Accordingly, from the viewpoint of the blend with the polyamide resin and the biocompatibility of the fiber, it is required to employ an ethylene-vinyl alcohol-series copolymer having an ethylene content of about 25 to 70% by mole.

As other components, there may be mentioned, for example, an additive such as a stabilizer (e.g., a heat stabilizer, an ultraviolet ray absorber, a light stabilizer, and an antioxidant), an organic or inorganic fine particle (e.g. , a crosslinking resin particle, a mineral particle, and a metal compound, or a ceramic particle), a coloring agent, an antistatic agent, aflameretardant, a plasticizer, a lubricant, a retardant for retarding the speed of crystallinity, or others. These additives may be used singly or in combination.

Further, in the present invention, a fiber constituting a fabric such as a clothing may include an conjugated fiber which conjugates the polymer A which may contain the polyamide and other components such as an additive with a polymer B other than the polymer A, in particular, in terms of hot water resistance, the preferred one includes a sheath-core structure conjugated fiber which comprises the polymer A as the sheath component and the polymer B as the core component.

(Polymer B)

The polymer B may include a conventional thermoplastic resin, for example, apolyolefin-seriesresin (e.g. , a polyC$_{2-4}$olefin-series resin such as a polyethylene or a polypropylene), an acrylic resin (e.g., an acrylonitrile-series resin having an acrylonitrile unit, such as an acrylonitrile-vinyl chloride copolymer), a vinyl-series resin (e.g. , a polyvinyl alcohol, and a vinyl chloride-series resin), a polyester-series resin (e.g., an aromatic polyester-series resin, and an aliphatic polyester-series resin), a polyamide-series resin (e.g., an aliphatic polyamide-series resin, an alicyclic polyamide-series resin, and an aromatic polyamide-series resin), a cellulose ester-series resin (e.g., a cellulose acetate), and others. These thermoplastic resins may be used singly or in combination. Among these thermoplastic resins, in view of heat resistance and dimensional stability, there is used a thermoplastic crystalline polymer having a melting point of not lower than 150°C (particularly about 170 to 270°C from the viewpoint of the spinnability of the conjugated fiber).

As such a thermoplastic crystalline polymer, there may be mentioned, for example, an aromatic polyester-series resin [e.g., a polyalkylene arylate-series resin having, as a main component, a C$_{2-4}$alkylene arylate unit such as ethylene terephthalate or butylene terephthalate (e.g., a polyC$_{2-4}$alkylene arylate such as a polyethylene terephthalate, a polytrimethylene terephthalate, a polybutylene terephthalate or a polyethylene naphthalate)], an aliphatic polyamide-series resin (e.g., a polyamide 6, a polyamide 66, a polyamide 11, a polyamide 12, a polyamide 610, and a polyamide 612), a polyolefin-series resin (e.g., a polypropylene-series resin such as a polypropylene), and others. Among these polymers, the commonly used one includes an aromatic polyester-series resin (e.g., a polyC$_{2-4}$alkylene arylate such as a polyethylene terephthalate or a polybutylene terephthalate), an aliphatic polyamide-series resin (e.g., a polyamide 6, and a polyamide 66), and others. The polymer B may contain an additive exemplified in the item of the polymer A.

(Characteristics of EVOH-series fiber)

It is preferred that the EVOH-series fiber is substantially free from a skin-irritative substance. In particular, from the viewpoint of non-irritability to patients with atopic dermatitis, it is preferred to use a low irritative surfactant as an oily substance to be used. Conventionally, the oily substances are selected in view of fiber productivity. However, such an oily substance is not always an oily substance having low irritability to human skin, and includes skin-irritative substances such as cationic and anionic surfactants. Therefore, in the present invention, as such an oily substance capable of alleviating itching of patients with atopic dermatitis, it is preferred to use a nonionic surfactant because of having a low irritation to skin. As the nonionic surfactant, there may be exemplified an ester-series nonionic surfactant, for example, a polyhydric alcohol fatty acid ester [e.g., an ester of a polyhydric alcohol with a fatty acid such as a (poly)glycerin, trimethylolpropane, propylene glycol, pentaerythritol, sorbitan, sorbitol or sucrose. These nonionic surfactants may be used singly or in combination. Among these nonionic surfactants, the preferred one includes an ester of glycerin and a $C_{8-24}$ fatty acid, such as a glycerin monostearate or glycerin trilaurate; an ester of sucrose and a $C_{8-24}$ fatty acid, such as sucrose monostearate; an ester of sorbitan and a $C_{8-24}$ fatty acid ester, such as sorbitan monolaurate or sorbitan monooleate. These nonionic surfactants usually adhere to a fiber surface, and the proportion of the nonionic surfactant is, for example, about 0.1 to 1.5% by mass, preferably about 0.2 to 1.2% by mass, and more preferably about 0.3 to 1% by mass relative to the fiber.

The EVOH-series fiber is required, in terms of biocompatibility, to be a fiber in which the polymer A covers not less than 70% of the fiber surface not only in the case where the fiber comprises the polymer A alone but also in the case where the fiber is a conjugated fiber. The coverage of the fiber surface with the polymer A is preferably not less than 80% (e.g., 80 to 100%), and more preferably not less than 90% (e.g., 90 to 100%). In the case where the fiber comprises the polymer A alone or is a conjugated fiber having the polymer A as the sheath component, it is usually regarded that 100% of the fiber surface is covered with the polymer A.

In the present invention, the molar proportion of the vinyl alcohol unit (the hydroxyl group amount) on the EVOH-series fiber surface is about 20 to 70% by mole, preferably about 21 to 50% by mole, and preferably about 22 to 45% by mole (particularly about 25 to 40% by mole). As described above, the OH group concentration on the fiber surface affects biocompatibility to the human body (i.e., alleviating or healing property of itching on the affected area of atopic dermatitis). Evaluation method of the biocompatibility to the human body is described below. Incidentally, both in the case where the OH group concentration is over 70% by mole and in the case where the OH group concentration is under 20% by mole, the fiber is insufficient in biocompatibility, whereas in the case where the OH group concentration is in the range of 20 to 70% by mole, the fiber realizes a favorable biocompatibility. The reason why the fiber in the above range realizes a good biocompatibility is not clear at this time, but such a biocompatibility is estimated to be caused by a specific molecular structure of the ethylene-vinyl alcohol-series copolymer used in the present invention.

The human skin exists on an appropriate balance between hydrophilicity and hydrophobicity, and the existence of water molecule having a specific binding force is thought to be important for the human skin. Since the ethylene-vinyl alcohol-series copolymer used in the present invention comprises a random copolymer having an ethylene unit as the hydrophobic unit and a vinyl alcohol unit as the hydrophilic unit, and forms a microphase-separated structure having a specific hydrophilicity and hydrophobicity in a specific copolymerization (content) range and fiber-forming condition, the ethylene-vinyl alcohol-series copolymer has a structure having both water affinity and hydrophobicity, which resembles to the human skin surface layer (epidermal layer). As a result, it is estimated that such an ethylene-vinyl alcohol-series copolymer is capable of forming a fiber having compatibility with the human body. The present invention is the first to find that the OH group concentration on the fiber surface and the given copolymerizable component play an important role on compatibility of the synthetic fiber with the human body, which compatibility affects atopic dermatitis.

The OH group concentration on the fiber surface is determined in the following manner. Even if the ethylene-vinyl alcohol-series copolymer simply covers the fiber surface, such a fiber is not always to have specific OH group concentrations defined in the present invention. That is, in the case where the OH groups on the fiber surface are replaced with a group other than OH group by acetalization or formalization, depending on the acetalization or formalization degree, such a fiber does not satisfy the OH group concentration on the fiber surface defined in the present invention. Basically, the OH groups of the polymer A covering the fiber surface preferably remain in the unreacted state on the fiber surface. The molar proportion of the vinyl alcohol unit on the EVOH-series fiber surface can be mainly controlled by the ethylene content or the crosslinking degree with acetalization.

The measurement of the OH group concentration on the fiber surface was carried out with AXIS-HSi manufactured by Shimadzu Corporation. The conditions of measurement are as follows: X-ray excitation condition: 15 kv - 15 mA, and pressure: $1 \times 10^{-7}$ Pa. In the measurement of the hydroxyl group concentration, the fiber was subjected to a chemical labeling procedure with trifluoroacetic acid, and the concentration was calculated from changes in spectrum between before and after labeling.

The biocompatibility of the fiber to the human body is evaluated by a user test on patients with atopic dermatitis who actually wear a clothing next to the skin. In the evaluation method by the user test, the patients with atopic dermatitis wore a clothing (T-shirt) of the present invention during daytime and nightclothes of the present invention while sleeping for 4 consecutive weeks each in summer and winter, and did an evaluation. As the evaluation, the daily VAS evaluation of itching degree by the trier [deciding an initial itching degree on a scale of one to ten at the starting of the test (or before starting), then evaluating the itching degree as increase or decrease from the initial itching degree] as well as the evaluation of the degree of scratched lesion by a medical doctor (doctor in attendance) on a scale of one to four were conducted. In the case of wearing the clothing of the present invention, an amazingly significant healing (curative) effect was recognized both in summer and winter seasons. In particular, it has been said until now that the curative effect is difficult to be achieved in winter because of deterioration in barrier property of the human skin surface. The clothing of the present invention is the first one having a curative effect on patients with atopic dermatitis who wear the clothing in winter. The details of the test are described in the after-mentioned Examples.

In the present invention, the fineness of the fiber constituting the clothing is also important. A fabric such as a clothing made from a fiber having too coarse fineness causes increasing irritation to the affected area of atopic dermatitis. Concretely, the fineness of the EVOH-series fiber is not more than 5 dtex (e.g., about 0.1 to 5 dtex), and the fiber having a fineness of over 5 dtex causes serious irritation to the affected area of atopic dermatitis and cannot be applied to a fabric for atopy patients. The fineness of the fiber is preferably not more than 4.5 dtex (e.g., about 0.2 to 4.5 dtex), more preferably not more than 4 dtex (e.g., about 0.2 to 4 dtex), and particularly about 0.5 to 4 dtex.

Further, in the present invention, the Young's modulus of the fiber is mentioned as one of the important aspects of the fiber. The Young's modulus of the EVOH-series fiber is not more than 60 cN/dtex (e. g. , about 3 to 60 cN/dtex), and the fiber having a Young's modulus of over 60 cN/dtex becomes inflexible, resulting in causing irritation to the affected area of atopic dermatitis. The Young's modulus of the fiber is preferably not more than 55 cN/dtex (e.g., about 5 to 55 cN/dtex), and more preferably about 10 to 55 cN/dtex. The Young's modulus may be adjusted by using a resin having a low Young's modulus as the polymer B constituting the fiber or by lowering a draw ratio of the fiber.

(Sheath-core structure conjugated fiber)

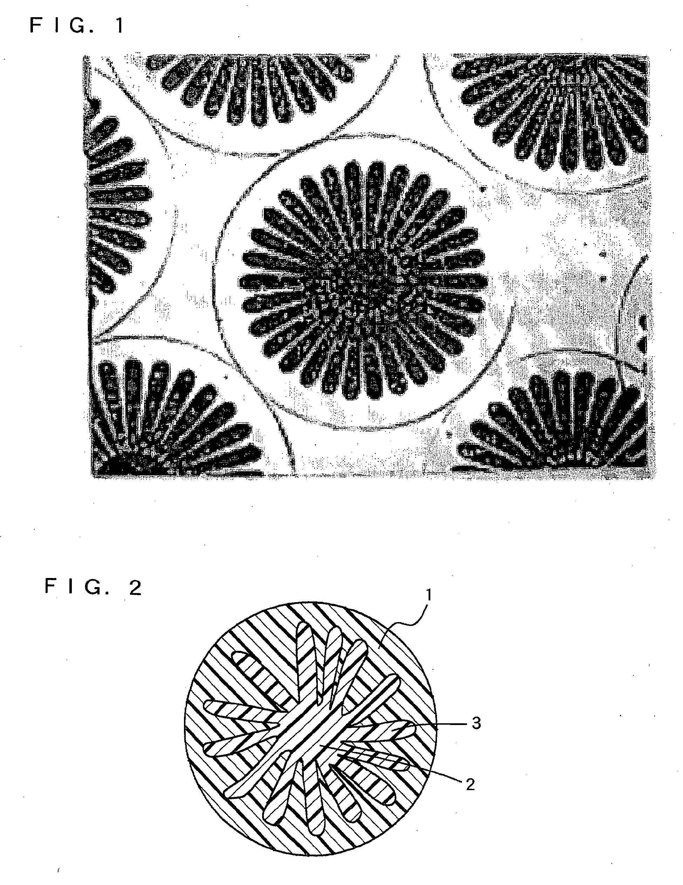

The EVOH-series fiber having such a property, from the viewpoint of durability or others, preferably includes the above-mentioned sheath-core structure conjugated fiber (a fiber comprising the polymer A as the sheath component and the polymer B as the core component). Thereinafter, the sheath-core structure conjugated fiber is illustrated in detail. In particular, in the present invention, the fiber preferably comprises a core having a cross-sectional structure having protrusions of not less than 4 in a perpendicular direction to the longitudinal direction of the fiber. Fig.1 shows a cross-sectional micrograph of one embodiment of such a sheath-core structure conjugated fiber. As apparent from the cross-sectional structure of Fig. 1, the fiber comprises a sheath comprising the polymer A and a core surrounded by the sheath, which comprises the polymer B. The core has a cross-sectional structure having 30 protrusions (tongue-like or leaf-like protrusions) which radially extend. Since such a fiber comprising the core with protrusions rarely causes separation between core and sheath, the fiber surface has few exposures of the core due to the core separation. Accordingly, such a fiber hardly causes irritation to the affected area of atopic dermatitis.

The core may have a shape in which a plurality of protrusions extend in a radial direction thereof from an appropriate portion(s) of the core (e.g., biased portion from the axial center, particularly the axial center or the midpoint), or may have a shape in which protrusions extend from an appropriate portion(s) of the core (e.g., the axial center or the midpoint) at regular and equally spaced intervals or irregular and randomized intervals in a circumferential direction thereof. The core usually has a plurality of protrusions (or out-thrusts) extending in a radial direction thereof from the axial center. The cross-sectional structure of the protrusions (or out-thrusts) may be in a quadrate form such as a square form or a rectangular form, an elliptical form, or an irregular form such as a branched form, and the aspect ratio of the projection (protrusion) [the ratio of the length (altitude) stuck out from the core relative to the width (base)] [altitude/base] is, for example, about 1/1 to 100/1, preferably about 2/1 to 50/1, and more preferably about 3/1 to 30/1 (particularly about 4/1 to 10/1). The protrusion having such an aspect ratio improves adhesion between core and sheath. Incidentally, it is not necessary that each of the protrusions has the same size or same shape. That is, the altitude of each protrusion may be same with or different from each other.

The number of protrusions is not particularly limited to a specific one as far as being not less than 4, and is preferably not less than 7 (e.g., about 7 to 100), more preferably not less than 10 (e.g., about 10 to 80), and further preferably not less than 20 (e.g., about 20 to 60). In the case where the number of protrusions is small, the core is liable to separate from the sheath. The upper value of the number of protrusions is not limited to a specific one, but in practical, 100 is the limit value from the standpoint of industrial production because of too compricated spinning nozzle structure.

Further, it is preferred that the ratio of the peripheral length of the core (L2) relative to the peripheral length of the conjugated fiber (L1) satisfies the following formula (1):

$$1.6 \leq X/C \quad (1)$$

wherein, X represents ratio (L2/L1) of the peripheral length of the core (L2) relative to the peripheral length of the conjugated fiber (L1), and C represents the conjugated ratio (mass ratio) of the core relative to the conjugated fiber when the mass of the conjugated fiber is taken as 1.

The ratio "X" may change depending on the conjugated ratio of the core, and the value "X/C" is preferably not less than 1.6 (e.g., about 1.6 to 30), more preferably not less than 2 (e.g., about 2 to 25), and further preferably not less than 2.5 (e.g., about 2.5 to 20). Too small X/C value lowers adhesion between the core and the sheath. On the contrary, the conjugated fiber having the X/C value of over 30 is difficult to commercially produce stably.

Accordingly, to take the case where the conjugated mass ratio of the core component relative to the sheath component [(core component) : (sheath component)] is about 60:40 to 40:60 (e.g. , 50:50) as an example, the ratio L2/L1 is preferably not less than 0.8 (e.g., about 0.8 to 15), more preferably not less than 1.2 (e.g., about 1.2 to 12), and more preferably not less than 1.25 (e.g., about 1.25 to 10). Too small L2/L1 ratio lowers adhesion between the core and the sheath. Contrarily, the conjugated fiber having a complicated interface with the ratio L2/L1 of over 15 is hard to commercially produce stably.

The fiber having a ratio "L2/L1" satisfying the above formula (1) effectively avoids interfacial separation of the core from the sheath (abruption in the interface between the core and the sheath). As the reason why the interfacial separation is avoided, there is estimated the increase of the adhesive surface area between the conjugated components as well as the anchoring effect of protrusions formed by the B component. Then, in the case where the interfacial separation can be avoided, as described above, the fiber does not have the core exporsure, and is capable of preventing the affected area of atopy from irritation.

Further, in the present invention, the space between adjacent protrusions is preferably not more than 2 $\mu$m (e.g. , about 0.1 to 2 $\mu$m, and particularly about 0.5 to 2 $\mu$m) in view of avoidance of the separation. Incidentally, in the present invention, the space between adjacent protrusions means the average girth of the sheath component.

It is not necessary to cover 100% surface of the conjugated fiber with the polymer A, but the more the degree of exposure of the polymer B on the surface is, the more the fiber causes irritation to the affected area of atopic dermatitis. Therefore, it is necessary that not less than 70% (e.g. , about 70 to 100%) of the surface of the conjugated fiber is covered with the polymer A, and the covered area with the polymer A on the surface of the conjugated fiber is preferably not less than 80% (e.g., about 80 to 100%), and more preferably not less than 90% (e.g., about 90 to 100%).

The cross-sectional structure of the whole fiber including the sheath is not limited to a specific one, and may include a round form as shown in Fig. 1, in addition, modified (irregular or deformed) cross-section forms, for example, a polygonal form such as a trigonal to octagonal form, a humilis or elliptical form, a T-shaped form, a Y-shaped form, a C-shaped form, and others.

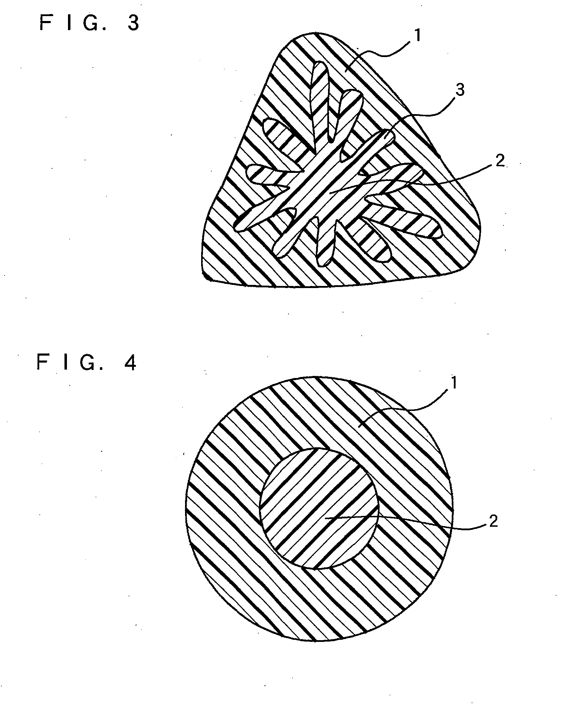

Figs. 2 to 4 show a schematic cross-sectional view illustrating other embodiments of the sheath-core structure conjugated fibers of the present invention. The fiber shown in Fig. 2 is provided with a sheath 1 with a cross-sectional circular form, and a core 2 surrounded by the sheath 1, and the core 2 has 14 pieces of leaf-like protrusions 3 extended in a circumferential direction thereof in a regular or irregular space from the core 2, and each protrusion 3 extends unradially (irregularly against the radial direction) from the center of the core 2. The fiber shown in Fig. 3 has a cross-section structure of a trigonal form, and is the same fiber with the fiber shown in Fig. 2 except that the core 2 has 11 pieces of protrusions 3. In the fiber shown in Fig. 4, the sheath 1 and the core 2 surrounded by the sheath 1, both having cross-sectional circular forms , are coaxially formed together. In comparison with fibers shown in Figs. 2 and 3, the fiber shown in Fig. 4 is liable to separate between the sheath 1 and the core 2 because of no protrusion in the core.

The conjugated ratio of the sheath component relative to the core component can be selected, in terms of mass ratio, for example, the range of 90:10 to 10:90, and is preferably about 80:20 to 20:80, and more preferably about 70: 30 to 30:70. In the case where the sheath component has too small ratio, it is difficult to completely (entirely) cover the fiber surface with the sheath component, and the fiber is not favorable to patients with atopic dermatitis.

The process for producing such a sheath-core structure conjugated fiber is not limited to a specific one as far as the process is capable of producing the conjugated fiber satisfying the requirement defined in the present invention, and the preferred process comprises, on the occasion of introduction of a conjugated stream of the polymer A and the polymer B into a nozzle inlet with a multi-component (bi-component) fiber spinning machine, allowing to the polymer B to flow from a flow-dividing plate provided with fine holes as many as the number of core protrusions, introducing the conjugated stream of the polymer A and the polymer B toward the center of the nozzle inlet with the whole stream of the polymer B flown from each fine hole surrounded with the polymer A, and discharging the conjugated stream from the nozzle. Moreover, as the spinning and drawing method, there may be adopted an arbitrary method such as a method comprising melt-spinning a yarn at a low or middle speed and drawing the spun yarn; a direct spinning and drawing method at a high-speed; or a method comprising simultaneously or successively drawing and false-twisting a spun yarn. By varying the conditions of spinning and/or drawing, the fineness of the fiber can be adjusted in the above-described range.

In the present invention, from the viewpoint of preventing the affected area of atopic dermatitis from the irritation, the EVOH-series fiber (the fiber comprising an ethylene-vinyl alcohol-series copolymer, or the sheath-core structure conjugated fiber comprising the copolymer as the sheath component) is preferably employed as a filament (continuous fiber). That is, in the case of using a short fiber (staple fiber) as a spun fiber to form a fabric (e.g., a clothing), cut ends of the fiber irritate the affected area of atopic dermatitis, resulting in effect decrease.

Conventionally, short fibers have been generally used for the clothing. By means of the short fiber, a lot of fiber ends are protruded from the fiber surface, and the fiber ends give the clothing the bulkiness as well as warmth retaining property. In the case of using such a fiber for the fabric in atopy patients, however, the fiber ends which exist in the conventional clothing are not preferred in the conventional clothing because the fiber ends causes irritation to the affected area. Accordingly, in the present invention, it is preferred to use a continuous fiber (multifilament yarn) scarcely having fiber ends in terms of low skin irritation.

The whole fineness of the multifilament yarn is preferably in the range of 40 to 160 dtex. With too thin (tenuous) multifilament yarn, it is difficult to industrially produce a textile (or woven or knitted product) . On the contrary, too thick filament deteriorates in hand touchiness of the fabric and as a result causes irritation to the affected area of atopy. More preferably, for example, the multifilament for a knitted fabric has a fineness of 60 to 100 dtex and the multifilament for a woven fabric has a fineness of 60 to 120 dtex. The multifilament yarn is usually a multifilament which comprises an EVOH-series multifilament alone, and may be a multifilament which comprises a multifilament other than the EVOH-series multifilament (e.g. , a natural fiber, a semisynthetic fiber, and a synthetic fiber) as the core yarn and the EVOH-series multifilament as the side yarn around the core yarn.

Thus obtained EVOH-series fiber is processed to a yarn (preferably a multifilament yarn) and the yarn is processed to a fabric for patients with atopic dermatitis. The false-twisting of the yarn used for a fabric (particularly, a clothing) usually has a crimp number (the false-twisting number) of around 2000 T/m, but the false-twisting of the yarn in the present invention is preferably a low crimp such as the false-twisting number of about 500 to 1500 T/m (preferably about 700 to 1450 T/m, and more preferably about 1000 to 1400 T/m) . According to such a false-twisting, the yarn is flattened and is able to have a soft skin touchiness and suppress skin irritation. Incidentally, in the false-twisting, the S twist and Z twist are alternatively conducted. Therefore, the number of crimp here is a total twisting number of S and Z twists. Too small false-twisting number lowers stretching property of the fabric, thereby the fabric becomes unsuitable for clothing such as an underwear. On the contrary, too large false-twisting number makes feel against the skin rough, and such a fiber causes stronger skin irritation.

Further, in the case where such a false-twisted fiber is processed to a fabric such as a woven or knitted fabric, by allowing the false-twisted fiber to be additionally twisted (i.e., actually twisted), although such an additional twisting is not conducting in the usual processing, the fiber achieves to have a good air permeability and refreshing (or cooling) property, and further ensures to alleviate skin irritation. In usual, the multifilament yarn used in the textile is actually twisted to form a yarn (strand) with doubling 2 to 3 multifilament yarns. In the present invention, however, it is preferred to twist one piece of the false-twisted yarn without doubling. In the case of doubling a plurality of yarns, the doubled yarn becomes stiff, resulting in undesirable irritation to the affected area of atopy.

The number of additional twisting is desired to be about 500 to 1200 T/m (preferably about 550 to 1100 T/m, and more preferably about 600 to 1000 T/m). Such an additional twist is expected to realize not only a alleviation of skin irritation but also a cooling effect. That is, by additional twisting, the air content of the fabric becomes small, and the maximum heat transferring quantity (Qmax) increases (e.g., the Qmax is $0.22J/m^2$ to $0.24J/m^2$). Thereby the fabric is capable of effectively transferring heat. The patients with atopic dermatitis are liable to increase skin temperature due to disturbance of sweating and easily cause itching or others. Therefore, since the fabric having a large Qmax is expected to have a cooling effect in which the heat is effectively removed from the skin surface and increase of body temperature is inhibited, such a fabric is a preferred material for patients with atopic dermatitis. Too little actual twisting (additional twisting) lowers cooling effect as well as refreshing property of the fabric. Contrarily, too much actual twisting is liable to cause uneven structure to the fabric, and such a fabric tends to generate point-contact with skin. Such a point-contact sometimes causes irritation to the skin.

The fabric is a nonwoven or woven fabric comprising the above-mentioned EVOH-series fiber, and usually a textile. As the textile, there may be mentioned, for example, a woven fabric such as a plain weave, a diagonal weave, or a satin weave; a knitted fabric such as a plain stitch, a rib stitch, a purl stitch, a chain stitch, a denbigh stitch, a plain cord stitch, an inlay stitch, or an atlas stitch; and others. Further, the fabric may be obtained by a combined (mixed) weave or knit using the EVOH-series fiber and a fiber other than the EVOH-series fiber (e.g., a synthetic fiber, a semisynthetic fiber, and a natural fiber). In terms of biocompatibility, the proportion of the other fiber is not more than 50% by mass, preferably not more than 30% by mass, and more preferably not more than 10% by mass. In particular, in the portion which comes in contact with the affected area, the EVOH-series fiber usually covers 100% by mass of the portion.

Such a fabric has low skin irritation and excels in biocompatibility. In particular, since the fabric ensures to alleviate the symptom (e.g., itching) of the affected area of patients with atopic dermatitis, the fabric is suitably used for healing the scratched lesion accompanied with itching. Therefore, the fabric of the present invention is used for contacting with the affected area of patients with atopic dermatitis. That is, the fabric of the present invention needs to have the EVOH-series fiber in the direct contact area with the affected area of atopic dermatitis.

The fabric of the present invention may be used as a material for a clothing [e.g., an underwear or an underclothing (e.g., a shirt, underpants or panties, a brassier, a chemise, a camisole or tank top, a corset or girdle, a combination, drawers, a slip, and a petticoat), a swimming suit (e.g., a bikini, a tank suit, and swimming shorts), a shirt (e.g., a T-shirt, a white shirt (dress shirt), a sport shirt, a polo shirt, and a sweatshirt), clothes (e.g., trousers or pants, and a skirt), a sleeping wear (e.g., nightclothes or pajamas, and a negligee), a hosiery (e.g., socks, stockings, and a pantyhose), and gloves or mittens]; a medical or sanitary material (e.g., a menstrual sanitary product, a diaper, a disposable diaper, a diaper liner, a mask, a towel, a sheet, a gauze, a bandage, a gown for medical application , a cap, an operating gown, and a surgical tape); a cosmetic supply (e.g., a cosmetic (skin-care) sheet such as a cleansing sheet, a sweat-removing sheet or an oil-blotting sheet, and a puff) ; commodities [e.g., bedclothes (e.g., a pillow, a pillow cover, a comforter or mattress, a cover for comforter or mattress, and a sheet), a cushion cover, a table cloth, a handkerchief, a washcloth, a towel, and a pre-moistened wipe (wet tissue)]; and others. In these applications, the fabric of the present invention is used for a surface member for contact with at least the affected area. Therefore, the fabric of the present invention is not limited to a specific one such as the above-exemplified matters, as far as the fabric constitutes a product which directly comes in contact with human skin. Incidentally, the surface member includes not only fiber products the surface of which is covered with the fabric of the present invention but also fiber products comprising the fabric of the present invention alone.

Among these applications, the preferred one includes an application in which the fabric frequently coming in contact with the skin, for example, a clothing such as an underwear, a swimming suit, a shirt, a sleeping wear, a hosiery, or gloves or mittens; a cosmetic sheet such as a cleansing sheet, a sweat-removing sheet or an oil-blotting sheet; particularly an underwear such as a shirt or underpants or panties, and a cosmetic sheet. For example, in the case of the underwear, it is preferred that all of the area where comes in contact with the human body comprises the EVOH-series fiber. Moreover, in the case of the clothing for wearing over the underwear, it is preferred that the areawhere does not come in contact with the underwear (e.g., neck, arm or leg part) and comes in contact with the human skin comprises the EVOH-series fiber. That is, the clothing of the present invention may comprise the EVOH-series fiber as the surface or area (or part) contacting with the human skin and a fiber other than the EVOH-series fiber (e.g., a natural fiber, a semisynthetic fiber, and a synthetic fiber) as other surface(s) or area(s) (or part(s)). Further, it is not necessary that all of the area where comes in contact with the skin comprises the EVOH-series fiber, and the area where comes in contact with the affected area of atopic dermatitis may comprise the EVOH-series fiber.

Furthermore, in the clothing (particularly underwear) of the present invention, from the viewpoint of alleviating the itching of the atopy patients, it is preferred that a surface which comes in contact with the skin comprises at least the EVOH-series fiber and an opposite surface thereof (that is, the surface which does not come in contact with the human body) comprises at least a cellulose-series fiber. Moreover, for example, each surface may be covered with the EVOH-series fiber or cellulose-series fiber. Since long remanence of the sweat from the human body on the skin surface increases itching, if the surface which is opposite to the surface contacting with the human body comprises the cellulose-series fiber, the sweat smoothly transfers from the human body to the cellulose-series fiber layer, and is absorbed by the cellulose-series fiber layer. Further, since the layer comprising the EVOH-series fiber exists between the sweat-containing cellulose fiber layer and the human body, the sweat-absorbed clothing scarcely adheres to the human body, and as the result, the clothing rarely causes irritation to the affected area of atopy. The illustrative examples of the cellulose-series fiber may include a cotton, a hemp, a rayon (e.g., a viscose rayon and a Bemberg rayon), a lyocell, and others.

INDUSTRIAL APPLICABILITY

The fabric of the present invention is useful for a clothing such as an underwear, a T-shirts, nightclothes, a negligee, a hosiery, a pantyhose, gloves or mittens; a sanitary material such as a menstrual sanitary product, a diaper liner or a disposable diaper; a fiber product for applying or removing cosmetics; and the like.

EXAMPLES

Hereinafter, the following examples are intended to describe this invention in further detail and should by no means be interpreted as defining the scope of the invention. Incidentally, unless otherwise indicated, "%" or "part(s) " in Examples indicates the proportion by mass.

Example 1 and Comparative Examples 4 and 5

Ethylene and vinyl acetate were allowed to a radical polymerization to prepare a random copolymer having an ethylene content of 44% by mole, subsequently the random copolymer was saponified with caustic soda to give a saponification product of the ethylene-vinyl acetate copolymer, i.e., an EVOH having a saponification degree of not less than 99% by mole. With using thus obtained polymer as the polymer A (for forming the sheath) and a polyethylene terephthalate (PET) as the polymer B (for forming the core), melt spinning of the multi-component was conducted in the conjugated ratio of the polymer A relative to the polymer B of 50:50, at a spinning temperature of 285°C, and a reeling speed of 3500 m/min. to give a conjugated filament fiber having a cross-sectional structure of each fiber shown in Fig. 1 [a multifilament comprising 24 filaments and having a whole fineness of 83 dtex (hereinafter mentioned as "83 dtex/24 filament")]. Prior to preparation of this fiber, a wet pellet used for the polymer A was washed with a large amount of pure aqueous solution containing acetic acid, followed by washing with an excessively large amount of purified water alone, so as to remove alkali metals and alkaline earth metals to give a washed pellet having contents of alkali metals and alkaline earth metals of 1. 2 ppm and 0. 8 ppm, respectively. Moreover, sorbitanmonolaurate as the oily substance adhered to the surface of the conjugated fiber.

The ratio (L1/L2) of the peripheral length of the conjugated fiber (L1) relative to the peripheral length of the polymer B (L2) was 4.0 (X/C=8.0), and the conjugated fiber had a strength of 3.2 cN/dtex, and a Young's modulus of 40 cN/dtex. Thus obtained multifilament yarn was false-twisted at 1300 T/m in a conventional manner, and further actually twisted at 800 T/m with a double twister. Then, a heat set for finishing twist was conducted at 110°C for 20 minutes under a reduced pressure.

With the resultant multifilament yarn, an interlock knitted fabric having a gauge of 28 was produced. The OH group concentration on the fiber surface was 28.9% by mole, which was measured with AXIS-HSi manufactured by Shimadzu Corporation. The greige was sewn in a T-shirt shape, and used as a test clothing for daytime underwear. On the contrary, nightclothes comprising thus obtained knitted fabric as an inner fabric and a cotton as an outer fabric were sewn and used as a test clothing for nighttime pajamas.

Test method

Subjects: 13 male and female patients with atopic dermatitis of 7-year to 42-year old having mild to middle itching. Wearing clothing: During the daytime, the T-shirt comprising the fiber obtained in Example 1.

During the nighttime, the nightclothes comprising the fiber as the inner surface and a cotton as the outer surface. Wearing time: 4 weeks both in midsummer and midwinter.

Evaluation method: The patients recorded daily itching degree with keeping the score [VAS evaluation on a scale of one to ten].

The medical doctor evaluated the degree of scratched lesion on a scale of one to four.

The patients described the itching degree before and after wearing.

The results of the wearing test in the summer revealed that the average VAS score of itching was changed from 4.96 before starting the test to 3.76 at 2 weeks after wearing and to 3.35 at 4 weeks after wearing, and that the degree of scratched lesion was decreased from 2.75 before starting the test to 1.92 at 4 weeks after wearing. Further, for curing effect was recognized in 8 patients out of 13 patients (improvement ratio of 62%). Contrarily, the results of the wearing test in the winter revealed that the average VAS score of 13 patients was changed from 4.15 before starting the test to 2.95 at 2 weeks after wearing and to 3.00 at 4 weeks after wearing, and that the degree of scratched lesion was decreased from 2 . 15 before starting the test to 1.54 at 4 weeks after wearing. Further, the curing effect was recognized in 11 patients out of 13 patients (improvement ratio of 85%).

Meanwhile, as Comparative Example 4, a multifilament yarn (84 dtex/ 36 filament) of a regular polyester (PET) was prepared in a conventional manner, and a knitted fabric was produced with the multifilament yarn in the same manner with Example 1, and the evaluation was carried out in the same manner as Example 1. Likewise, as Comparative Example 5, a continuous fiber yarn (77 dtex/24 filament) comprising a polyamide 6 was prepared in a conventional manner, and a knitted fabric was produced with the continuous fiber yarn in the same manner with Example 1, and evaluation was carried out in the same manner as Example 1. Neither Comparative Examples 4 nor 5 had an effect for alleviating itching of the patients with atopic dermatitis. The results were shown in Table 1.

Example 2

Example 2 is an embodiment in which a fiber was obtained with a commercially available polyamide 6 as the polymer B. That is, except for using the polyamide 6 as the polymer B, a continuous fiber was prepared in the similar manner with Example 1, and a T-shirt shaped underwear was obtained from a knitted fabric comprising the continuous fiber. Then, the wearing test was conducted. The results were shown in Table 1. The results in Table 1 show that the clothing was excellent in the effect for alleviating itching of the patients with atopic dermatitis.

Example 3

To the same ethylene-vinyl alcohol-series copolymer with that of Example 1, a polyamide resin synthesized in the production process shown below was melt-mixed in the proportion of 20%, and the mixture was extruded, and the resultant was used as the polymer A. As the polymer B, a commercially available polyamide 6 was used and a sheath-core structure conjugated fiber (core: sheath= 1:1) comprising the polymer A as the sheath component and the polymer B as the core component was discharged from a spinneret at a spinning temperature of 285°C and the spun filament was reeled at a reeling speed of 1000 m/min. and piled up in a can. Thereafter, the obtained fiber tow was drawn, twisted and cut in the common manner to give a staple fiber (single fiber fineness of 1.2 dtex, and fiber length of 38 mm) having a cross sectional combined shape shown in Fig. 1. In the polymer A, the average size of islands of the polyamide resin was 70 nm and the number of islands was about 50 per $\mu m^2$.

The Young's modulus of a raw stock comprising the staple fiber was 15 cN/dtex. The raw stock (70%) and a cotton (30%) were blended, and therefrom was prepared a spun yarn having a cotton count of 60. From thus obtained spun yarn, an interlock knitted fabric having a gauge of 28 was formed and dyed in the following condition to produce a T-shirt shaped underwear. Then the same wearing test with Example 1 was conducted. The results are shown in Table 1.

Moreover, a web was formed from the fiber of this Example, and the web was needle-punched and hydroentangled to give a nonwoven fabric. Thereafter, this nonwoven fabric was employed as a wiping material which comprised a surface material constituting an absorbent cotton layer, and the wiping material was used for wiping a cosmetic by three female patients with atopic dermatitis. As a result, the wiping material received recognition that itching was alleviated. Further, the fiber of this Example was knitted for producing stockings, and the stockings were used by three female patients with atopic dermatitis. Then the stockings received recognition that the stockings were comfortable due to alleviation of itching.

<Production of synthetic polyamide>

Into a pressure reactor (30 liter), were charged ε-caprolactam (10 kg) as a polyamide monomer, 1,6-hexanediamine (82 g) as a molecular weight adjusting agent, and water (1 kg), and the mixture were heated to 260°C with agitating, and pressurized up to 0.5 MPa. Thereafter, the pressure was released up to a normal pressure, and the mixture was polymerized at 260°C for 3 hours. At the end of polymerization, the reaction product was extruded in a strand shape, and the extruded matter was cooled and solidified, then cut to form a resin pellet of a polyamide 6. Thus obtained pellet was treated with hot water of 95°C, and dried to give a polyamide resin. The relative viscosity of the polyamide resin was 2.7 dl/g.

This polyamide resin was dry-blended with phthalic anhydride, and the blended matter was mixed and reacted at a temperature of 260°C with a biaxial extruder, and the reaction product was extruded in a strand shape and cut to obtain a terminal group-modified polyamide. The relative viscosity of the terminal group-modified polyamide was 2.61 dl/g; the amounts of the terminal amino group, the terminal carboxylic acid group, and the terminal phthalimide structure were 4 μeq. /g, 20 μeq. /g, and 77 μeq. /g, respectively; and the proportion of the cyclic phthalimide group in the total amount of the terminals was 76%.

Incidentally, in the polyamide resin, the relative viscosity, the terminal amino group amount, the terminal carboxylic acid amount, and the terminal imide structure amount were determined in the following manner.

<Relative viscosity (dl/g)>

The polyamide resin was dissolved in 97% concentrated sulfuric acid so as to have a concentration of 1 g/dl, and the viscosity of the solution was measured with Ubbelohde viscometer at 25°C.

<Terminal amino group amount (μeq./g)>

The polyamide resin was dissolved in phenol, and the solution was titrated with hydrochloric acid (0.05 N) to determine the terminal amino group amount. The amount was shown as the concentration of the terminal amino group per the mass of the polyamide resin.

<Terminal carboxylic acid amount (μeq./g)>

The polyamide resin was dissolved in benzyl alcohol, and the solution was titrated with an aqueous solution of potassium hydroxide (0.05 N) to determine the terminal carboxylic acid group amount. The amount was shown as the concentration of the terminal carboxyl group per the mass of the polyamide resin.

<Terminal imide structure amount (μeq./g)>

The polyamide resin was dissolved in deuteration hexafluoroisopropanol, and the solution was subjected to measure, with a 500MHz-NMR manufactured by JEOL Ltd., a peak area of hydrogen in the methylene group adjacent to the terminal imide structure. The peak area was converted into the terminal imide group concentration per the mass of the polyamide resin.

<Proportion of terminal imide structure>

The total amount of the above-mentioned terminal amino group, terminal carboxylic acid and terminal imide structure was computed, and the proportion of the terminal imide structure in the total amount was determined. Incidentally, "owf" means the proportion (weight ratio) of a chemical agent relative to an object fiber.

<Dyeing condition>

Multifix Blue N-FR (reactive dye) 2% owf
Manufacturer: Daiichikasei Co., Ltd
$Na_2SO_4$: 50 g/liter
Bath ratio = 1:50
In a bath filled with the above dyeing aqueous solution, an object material was immersed, and the bath was heated from 50°C to 98°C in 15 minutes, and the temperature of 98°C was kept for 20 minutes. Thereafter the temperature of the solution was lowered to 70°C over 10 minutes; to the solution was added $Na_2SO_4$ in the above ratio; the temperature of the mixture was maintained at 70°C for 30 minutes; and the temperature was subsequently decreased to finish the dyeing treatment. Subsequently, the dyed material was taken out from the bath and subjected to soaping for 20 minutes in hot water (95°C) containing "BISNOL RK" (an aliphatic nitrogen-containing compound, manufactured by Ipposha Oil Industries Co., Ltd) in the concentration of 1 g/liter. Finally, the soaped,material was subjected to a fixing treatment for 20 minutes in water of 50°C having a bath ratio of 1:50 and containing "DANFIX SC-8" (a quaternary ammonium salt-modified polymer, manufactured by Nitto Boseki Co., Ltd) in a concentration of 6% owf so as to complete the dyeing treatment.

Examples 4 to 5 and Comparative Examples 6 to 7

Except for using, as the polymer A, a saponification product of ethylene-vinyl acetate having a saponification degree of 99%, in which the ethylene content was changed as shown in Table 1, the same procedure was conducted with Example 1. The results are shown in Table 1. Examples 4 and 5 are the case in which the ethylene content in the polymer A was within the range defined in the present invention, and such cases satisfied the object of the present invention.
On the contrary, in Comparative Examples 6 and 7, since the ethylene content in the polymer A was out of the range defined in the present invention, the spinnability of the fiber was deficient. Moreover, since increase of the ethylene content in the polymer A enlarged hydrophobicity of the fiber and was attributed to the deterioration of the fiber in the affinity to the skin, the fiber caused severer irritation to the affected area of atopic dermatitis and was insufficient in effect.

Example 6

In the same manner with Example 1, was prepared a random copolymer having an ethylene content of 44% by mole, subsequently the random copolymer was saponified with caustic soda and pelletarized without washing to give an ethylene-vinyl alcohol copolymer having a saponification degree of not less than 99%. The impurity amount in the polymer (total of an alkali metal content and an alkaline earth metal content) was 390 ppm. This polymer as the polymer A and a polyethylene terephthalate as the polymer B were conjugately spun and drawn in the conjugated ratio of the polymer A relative to the polymer B (polymer A/polymer B) of 50/50 to form a multifilament (84dtex/24filament) . With thus obtained filament yarn, an interlock knitted fabric having a gauge of 28 was produced, and the knitted fabric was sewn in the same manner with Example 1 to form a T-shirt and nightclothes . Then the same evaluation method with Example 1 was conducted.
Improvement in atopic dermatitis was significantly recognized compared with Comparative Examples consisting of the polyester fiber, but the result was inferior to the results of the above all Examples.

Example 7

In the same manner with Example 1, a spun and drawn yarn (84dtex/24filament) was formed. In the process, an ionic surfactant such as a sodium polyoxyethylene stearyl ether sulfate, stearic acid amide amine, or stearylbetain was used as the oily substance. Then, with thus obtained yarn, a T-shirt and nightclothes were sewn and evaluated in the same manner with Example 1. Although the improvement in atopic dermatitis was observed, as with the above Example 6, the result was not good as the results of Examples 1 to 5.

Comparative Example 1

In the same manner with Example 1, a spun and drawn yarn (84dtex/24filament) was formed. Thus obtained continuous fiber was false-twisted at 120°C, and entwined in the cheese-like shape to form a cheese. With the cheese, the OH group on the surface of the ethylene-vinyl alcohol-series copolymer was acetalized in the following crosslinking condition in order to increase hot water resistance. With thus obtained filament yarn, an interlock knitted fabric having a gauge of 28 was produced, and the knitted fabric was sewn in the same manner with Example 1 to form a T-shirt and nightclothes. Then the same evaluation method with Example 1 was conducted. The results are shown in Table 1.

| | |
|---|---|
| <Crosslinking condition> | |
| <Bath formulation> | |
| Nonanedial | 7.5 g/liter |
| Sodium dodecylbenzenesulfonate | 0.5 g/liter |
| Nonionic surfactant | 0.5 g/liter |
| Hydrated maleic acid | 1.0 g/liter |
| <temperature x time> | 85°C x 40 min. |
| <Bath ratio>. | 1:10 |

Incidentally, the OH group concentration on the fiber surface was 18.0% by mole. According to the processing for the hot water resistance, the OH group concentration on the fiber surface became low, and the evaluation of objective biocompatibility was insufficient.

Comparative Example 2

The same procedure with Example 1 was conducted to give a fiber except that the fiber was melt spun at 300°C by using the same ethylene-vinyl alcohol-series copolymer with Example 1 as the polymer A and the following semiaromatic polyamide resin as the polymer B. A T-shirt and nightclothes were produced from the fiber, and subjected to the evaluation.

The semiaromatic polyamide used as the polymer B comprised 1,9-nonanediamine unit and 2-methyl-1,8-octanediamine unit as the diamine unit and terephthalic acid unit as the dicarboxylic acid unit. The molar ratio of the nonanediamine unit relative to the methyloctanediamine unit [(nonanediamine unit):(methyloctanediamine unit)] was 90:10.

The Young's modulus of the fiber was 80 cN/dtex. The results are shown in Table 1. Since the fiber had a large Young's modulus, the fiber causes severe irritation to the skin, and the result of the wearing evaluation was unsatisfactory.

Comparative Example 3

The same procedure with Example 1 was carried out except that the spun and drawn yarn was a multifilament of 84 dtex/ 16 filaments. The results are shown in Table 1. Since the yarn had a large single fiber fineness of 5.2 dtex, the yarn caused severe irritation to the skin, and hardly exercised the effect of itching alleviation on the patients with atopic dermatitis.

Incidentally, in the above Example, 100% of the fiber surface was covered with the EVOH-series fiber.

[Table 1]

Table 1

| Examples | A component | B component | OH group concentration of fiber surface (% by mole) | Young's modulus (cN/dtex) | single fiber fineness (dtex) | VAS score change of itching degree | | Score change of degree of scratched lesion | | Comments |
|---|---|---|---|---|---|---|---|---|---|---|
| | | | | | | Before starting | 4 weeks after | Before starting | 4 weeks after | |
| 1 | EVOH (44% by mole of ethylene) | PET | 28.9 | 45 | 3.4 | 4.96 | 3.35 | 2.75 | 1.92 | Filament |
| 2 | EVOH (44% by mole of ethylene) | Polyamide 6 | 29.1 | 35 | 3.4 | 4.98 | 3.25 | 2.80 | 1.90 | Filament |
| 3 | EVOH (44% by mole of ethylene) + Polyamide | Polyamide 6 | 27.5 | 15 | 1.2 | 4.95 | 3.40 | 2.80 | 1.95 | Staple |
| 4 | EVOH (35% by mole of ethylene) | PET | 30.5 | 48 | 3.4 | 4.95 | 3.20 | 2.75 | 1.85 | Filament |
| 5 | EVOH (60% by mole of ethylene) | PET | 20.5 | 42 | 3.4 | 4.98 | 3.50 | 2.78 | 2.00 | Filament |
| 6 | EVOH (44% by mole of ethylene) | PET | 28.9 | 45 | 3.4 | 4.95 | 4.55 | 2.80 | 2.45 | Filament (unwashed) |
| 7 | EVOH (44% by mole of ethylene) | PET | 28.9 | 45 | 3.4 | 4.95 | 4.40 | 2.80 | 2.30 | Filament (ionic oily substance) |

EP 1 764 430 A1

Table 1 (continued)

| Comparative Examples | A component | B component | OH group concentration of fiber surface (% by mole) | Young's modulus (cN/dtex) | single fiber fineness (dtex) | VAS score change of itching degree | | Score change of degree of scratched lesion | | Comments |
|---|---|---|---|---|---|---|---|---|---|---|
| | | | | | | Before starting | 4 weeks after | Before starting | 4 weeks after | |
| 1 | EVOH (44% by mole of ethylene) | PET | 18.0 | 45 | 3.4 | 4.95 | 4.80 | 2.75 | 2.70 | Acetalized |
| 2 | EVOH (44% by mole of ethylene) | Semiaromatic polyamide | 28.5 | 80 | 3.4 | 4.96 | 5.01 | 2.75 | 2.80 | Rigid core component |
| 3 | EVOH (44% by mole of ethylene) | PET | 28.0 | 55 | 5.2 | 4.95 | 4.70 | 2.76 | 2.65 | Thick fiber |
| 4 | PET | | — | 80 | 2.3 | 4.96 | 5.55 | 2.74 | 3.05 | No EVOH |
| 5 | Polyamide 6 | | — | 55 | 3.2 | 4.96 | 5.70 | 2.75 | 3.30 | No EVOH |
| 6 | EVOH (20% by mole of ethylene) | PET | — | — | — | — | — | — | — | Small ethylene amount |
| 7 | EVOH (80% by mole of ethylene) | PET | 12.5 | 38 | 3.4 | 4.96 | 4.61 | 2.75 | 2.62 | Too much ethylene amount |

**Claims**

1. A fabric for patients with atopic dermatitis, which is contactable with an affected area of patients with atopic dermatitis and comprises an ethylene-vinyl alcohol (EVOH)-series fiber comprising an ethylene-vinyl alcohol-series copolymer having an ethylene content of 25 to 70% by mole, in which the surface of the EVOH-series fiber is occupied by the ethylene-vinyl alcohol-series copolymer in a proportion of not less than 70%,
   wherein the proportion of the vinyl alcohol unit existing on the EVOH-series fiber surface is 20 to 70% by mole, the Young's modulus of the EVOH-series fiber is not more than 60 cN/dtex, and the single fiber fineness of the EVOH-series fiber is not more than 5 dtex.

2. A fabric according to claim 1, wherein the EVOH-series fiber is a sheath-core structure conjugated fiber comprising the ethylene-vinyl alcohol-series copolymer as the sheath component.

3. A fabric according to claim 2, wherein the EVOH-series fiber is a sheath-core structure conjugated fiber which comprises the ethylene-vinyl alcohol-series copolymer having an ethylene content of 25 to 70% by mole as the sheath component, and a crystalline thermoplastic resin having a melting point of not lower than 150°C as the core component.

4. A fabric according to claim 2, wherein the sheath-core structure conjugated fiber comprises a core having a cross-sectional structure having not less than 4 protrusions in a perpendicular direction to the longitudinal direction of the fiber and has a ratio of a peripheral length of the core (L2) relative to a peripheral length of the conjugated fiber (L1) satisfying the following formula (1):

$$1.6 \le X/C \quad (1)$$

wherein, X represents a ratio (L2/L1) of the peripheral length of the core (L2) relative to the peripheral length of the conjugated fiber (L1), and C represents the conjugated ratio (mass ratio) of the core relative to the conjugated fiber when the mass of the conjugated fiber is taken as 1.

5. A fabric according to claim 2, wherein the sheath component further comprises, in a proportion of 5 to 45% by mass relative to the sheath component, a polyamide resin whose terminal amino group is at least partly capped.

6. A fabric according to claim 1, wherein the EVOH-series fiber constitutes a multifilament yarn having a whole fineness of 40 to 160 dtex, the multifilament yarn is false-twisted, and the false-twisted yarn is further actual-twisted at a twist number of 500 to 1500 T/m.

7. A fabric according to claim 1, wherein the ethylene-vinyl alcohol-series copolymer contains an alkali metal ion and an alkaline earth metal ion in a proportion of not more than 100 ppm on the mass basis, respectively, and the EVOH-series fiber contains an oily substance comprising a nonionic surfactant.

8. A fabric according to claim 1, wherein an area contacting with an affected area of a wearer comprises the EVOH-series fiber.

9. A clothing for patients with atopic dermatitis, which comprises a fabric recited in claim 1.

10. A clothing according to claim 9, which is at least one member selected from the group consisting of an underwear, a shirt, a sleeping wear, a hosiery, gloves and a mask.

11. A clothing according to claim 9, which is an underwear for patients with atopic dermatitis, which comprises at least the EVOH-series fiber on the side contacting with skin of the patient and at least a cellulose-series fiber on the other side thereof.

12. A method for alleviating or healing a dermatitis symptom of patients with atopic dermatitis with the use of a fabric recited in claim 1.

F I G. 1

F I G. 2

F I G. 3

F I G. 4

<table>
<tr><td colspan="2"><b>INTERNATIONAL SEARCH REPORT</b></td><td>International application No.<br>PCT/JP2005/011753</td></tr>
</table>

| | |
|---|---|
| **INTERNATIONAL SEARCH REPORT** | International application No.<br>PCT/JP2005/011753 |

A. CLASSIFICATION OF SUBJECT MATTER
*D04B1/16* (2006.01), *A41B9/00* (2006.01), *11/00* (2006.01), *17/00* (2006.01), *A61L15/00* (2006.01), *D01F8/10* (2006.01), *D03D15/00* (2006.01)

According to International Patent Classification (IPC) or to both national classification and IPC

B. FIELDS SEARCHED

Minimum documentation searched (classification system followed by classification symbols)
*D04B1/16* (2006.01), *A41B9/00* (2006.01), *11/00* (2006.01), *17/00* (2006.01), *A61L15/00* (2006.01), *D01F8/10* (2006.01), *D03D15/00* (2006.01)

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho    1971-2005   Toroku Jitsuyo Shinan Koho   1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-306849 A  (Nippon Wishborn Corp.),<br>31 October, 2003 (31.10.03),<br>Claims<br>(Family: none) | 1-11 |
| A | JP 10-262858 A  (Kabushiki Kaisha Tosuko Chuo Kenkyusho),<br>06 October, 1998 (06.10.98),<br>Claims<br>(Family: none) | 1-11 |
| A | JP 10-110380 A  (Asahi Chemical Industry Co., Ltd.),<br>28 April, 1998 (28.04.98),<br>Claims<br>(Family: none) | 1-11 |

[X] Further documents are listed in the continuation of Box C.          [ ] See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>27 October, 2005 (27.10.05) | Date of mailing of the international search report<br>15 November, 2005 (15.11.05) |
| Name and mailing address of the ISA/<br>Japanese Patent Office | Authorized officer |
| Facsimile No. | Telephone No. |

Form PCT/ISA/210 (second sheet) (April 2005)

## INTERNATIONAL SEARCH REPORT

| International application No. |
| --- |
| PCT/JP2005/011753 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 3-213535 A  (Kuraray Co., Ltd.),<br>18 September, 1991 (18.09.91),<br>Claims<br>(Family: none) | 1-11 |

Form PCT/ISA/210 (continuation of second sheet) (April 2005)

**INTERNATIONAL SEARCH REPORT**

| International application No. |
|---|
| PCT/JP2005/011753 |

---

**Box No. II      Observations where certain claims were found unsearchable (Continuation of item 2 of first sheet)**

This international search report has not been established in respect of certain claims under Article 17(2)(a) for the following reasons:

1. [X] Claims Nos.: 12
   because they relate to subject matter not required to be searched by this Authority, namely:
   Claim 12 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

---

**Box No. III      Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant.  Consequently, this international search report is restricted to the invention first mentioned in the claims; it is covered by claims Nos.:

**Remark on Protest**      [ ] The additional search fees were accompanied by the applicant's protest and, where applicable, the payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8092820 A
- JP 10212456 A
- JP 2000044598 A

- JP 2003342835 A
- JP 3213535 A

**Non-patent literature cited in the description**

- The preparation and application of functional fibers from crab shell chitin. *Journal of Biotechnology,* 1999, vol. 70, 373-377
- Study on affectivity of a coefficient of variation of perspiration differential amount as an evaluation index of biocompatibility of various fibers. *Japanese Journal of Perspiration Research,* 2002, vol. 9 (2), 57-60

- Study on affectivity of a coefficient of variation of perspiration differential amount as an evaluation index of biocompatibility of various fibers. *Japanese Journal of Perspiration Research,* 2002, vol. 9 (2), 57-60